# EUROPEAN PATENT APPLICATION

(11) **EP 3 168 289 A1**
(43) Date of publication of application: **17.05.2017**
(21) Application number: 16197239.3
(22) Date of filing: 04.11.2016
(51) Int. Cl.: C12M 1/00

(54) **CELL CULTURE APPARATUS**

(30) Priority: 13.11.2015 JP 2015222546
(71) Applicant: Olympus Corporation, Hachioji-shi Tokyo 192-8507 (JP)
(72) Inventor: Kimura, Hiroyuki, Tokyo, 192-8507 (JP); Minami, Tatsuya, Tokyo, 192-8507 (JP); Makara, Yasunori, Tokyo, 192-8507 (JP)
(74) Representative: Schicker, Silvia

(57) **Abstract**

A cell culture apparatus (100) of the present invention includes: a body (1) including a space for accommodating a culture container (10), and an opening (1a) through which the culture container (10) is taken in and out; a door (2) for opening and closing the opening (1a); an optical detection means (3) that includes optical sensors (3a, 3b) for optically detecting an instruction to open and close the opening (1a) from a user, and converts the detection by the optical sensors (3a, 3b) to a signal and sends the signal; and a door open/close means (4) that receives the signal from the optical detection means (3) and controls open/close of the opening (1a) by the door (2).

## Description

### {Technical Field}

The present invention relates to a cell culture apparatus for culturing cells in a cell culture container, particularly to an incubator.

### {Background Art}

In recent years, with the development of the study of regenerative medicines, biopharmaceutics, and the like, a large-scale preparation of cells has been required. Cells are generally cultured in an incubator that can maintain conditions suitable for growth. To observe the states of cells regularly and exchange a culture medium, a culture container containing the cells and the culture medium needs to be taken out of the incubator and undergo work (see PTL 1, for example).

### {Citation List}

### {Patent Literature}

{PTL 1}
   Japanese Unexamined Patent Application, Publication No.2010-273603

### {Summary of Invention}

### {Technical Problem}

The operator needs to open and close the door of an incubator when taking a cell culture container in and out of the incubator, but with as little contact as possible in order to avoid a risk of contamination inside the culture container.

Besides, during work using a large amount of sample, the operator sometimes has difficulty opening and closing the door of the incubator with his hands full when taking the cell culture container in and out of the incubator.

In addition, for preparation of clinically-used cells, for example, work using a closed system with an isolator or the like is required, limiting a movable space for the operator and further increasing the load on the operator.

It is an object of the present invention, which has been made in this background, to provide a cell culture apparatus with a door that an operator can open and close without contact.

### {Solution to Problem}

To achieve this object, the present invention provides the following means.

One aspect of the present invention is a cell culture apparatus including: a body including a space for accommodating a culture container, and an opening through which the culture container is taken in and out; a door for opening and closing the opening; an optical detection means that includes an optical sensor for optically detecting an instruction to open and close the opening from an operator, and converts the detection by the optical sensor to a signal and sends the signal; and a door open/close means that receives the signal from the optical detection means and controls open/close of the opening by the door.

According to this aspect, the operator can open and close the door of the cell culture apparatus without contact, reducing a risk of contamination due to unnecessary contact. The operator can also take the sample in and out even with his hands full and the operability for the operator can be improved. In addition, the operator can be focused on the sample when taking the sample in and out and a shock on the sample can be therefore reduced, thereby reducing an impact on the culture medium and cells in the sample.

In this aspect, the optical detection means may include a first optical sensor that optically detects an instruction to open the opening, and a second optical sensor that optically detects an instruction to close the opening, the optical detection means may send a first signal for opening the opening in response to the detection by the first optical sensor and send a second signal for closing the opening in response to the detection by the second optical sensor, and the door open/close means may open the opening with the door upon reception of the first signal, and close the opening with the door upon reception of the second signal.

This allows the operator to speedily select open/close by selection between the two sensors.

In this aspect, the optical detection means may include a single optical sensor, the optical detection means may alternately send a first signal for opening the opening and a second signal for closing the opening each time the optical sensor performs detection, and the door open/close means may open the opening with the door upon reception of the first signal, and close the opening with the door upon reception of the second signal.

Hence, the operator only has to operate just one sensor and the operability can be improved.

In this aspect, the optical sensor may be an infrared sensor.

Another aspect of the present invention is a cell culture apparatus including: a body including a space for accommodating a culture container and an opening through which the culture container is taken in and out; a door for opening and closing the opening; a detection means that includes a pedal for detecting an instruction to open and close the opening from an operator, and converts the detection by the pedal to a signal and sends the signal; and a door open/close means that receives the signal from the detection means and controls open/close of the opening by the door.

According to this aspect, the operator can open and close the door without using his hands, reducing a risk of contamination. The operator can also take the sample in and out even with his hands full and the operability for the operator can be improved. In addition, the operator can be focused on the sample when taking the sample in and out and a shock on the sample can be therefore reduced, thereby reducing an impact on the culture medium and cells in the sample.

In this aspect, the detection means may include a first pedal for detecting an instruction to open the opening, and a second pedal for detecting an instruction to close the opening, the detection means may send a first signal for opening the opening in response to the detection by the first pedal, and a second signal for closing the opening in response to the detection by the second pedal, and the door open/close means may open the opening with the door upon reception of the first signal, and close the opening with the door upon reception of the second signal.

This allows the operator to speedily select open/close by selection between the two pedals.

In this aspect, the detection means may include a single pedal, the detection means may alternately send a first signal for opening the opening and a second signal for closing the opening each time the pedal performs detection, and the door open/close means may open the opening with the door upon reception of the first signal, and close the opening with the door upon reception of the second signal.

Hence, the operator only has to operate just one sensor and the operability can be improved.

Another aspect of the present invention is a cell culture apparatus comprising: a body including a space for accommodating a culture container, and an opening through which the culture container is taken in and out; a door for opening and closing the opening; an detection means that includes a sensor for detecting an instruction to open and close the opening from an operator, and converts the detection by the sensor to a signal and sends the signal; and a door open/close means that receives the signal from the detection means and controls open/close of the opening by the door.

### {Advantageous Effects of Invention}

According to the present invention, the operator can open and close the door of the cell culture apparatus without contact, reducing a risk of contamination due to unnecessary contact. The operator can easily open and close the door of the cell culture apparatus even with his hands full and the operability for the operator can be therefore improved. In addition, the operator can be focused on the culture container when taking the culture container in and out of the cell culture apparatus, and a shock on the culture container can be therefore reduced, thereby reducing an impact on the culture medium and cells in the culture container.

### {Brief Description of Drawings}

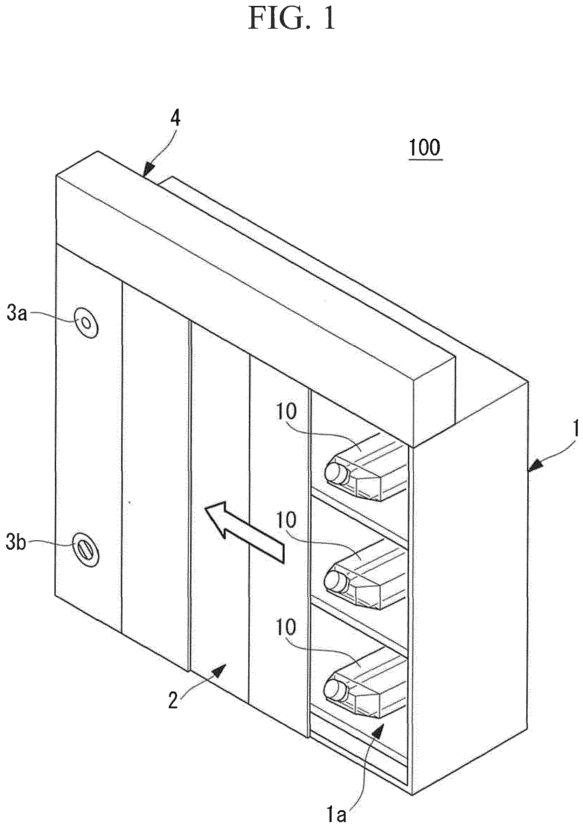

{Fig. 1}
   FIG. 1 is a diagram showing a schematic structure of a cell culture apparatus according to the first embodiment of the present invention.
{FIG. 2}
   FIG. 2 is a diagram showing a schematic structure of a cell culture apparatus according to the second embodiment of the present invention.
{FIG. 3A}
   FIG. 3A is a diagram (plan view) showing a schematic structure of a cell culture apparatus according to a modification of the first and second embodiments of the present invention.
{FIG. 3B}
   FIG. 3B is a diagram (plan view) showing a schematic structure of a cell culture apparatus according to another modification of the first and second embodiments of the present invention.
{FIG. 3C}
   FIG. 3C is a diagram (plan view) showing a schematic structure of a cell culture apparatus according to still another modification of the first and second embodiments of the present invention.

### {Description of Embodiments}

A cell culture apparatus according to an embodiment of the present invention will now be described with reference to the drawings.

### {First embodiment}

A cell culture apparatus according to the first embodiment of the present invention, which is an incubator 100 having the structure shown in FIG. 1, can maintain its inside at conditions (for example, temperature, humidity, and CO₂ concentration) suitable for cell culture and accommodate a culture container (cell culture container) 10 containing cells, thereby allowing for cell culture.

The body 1 having a space for accommodating the culture container 10 includes an opening 1a through which the culture container 10 is taken in and out. The opening 1a is provided with an openable door 2. Opening the door 2 exposes the internal space of the body 1 through the opening 1a, thereby providing a state in which the culture container 10 can be taken in and out. Closing the door 2 shields the internal space of the body 1, thereby maintaining its inside at conditions (for example, temperature, humidity, and CO₂ concentration) suitable for cell culture.

The body 1 may have, for example, a box structure with one face opened.

The incubator 100 includes an optical detection means 3 including an optical sensor. When the operator holds his hand or the like close to but not in contact with the optical sensor, the optical detection means 3 detects it and converts it to a signal (e.g., an electrical signal) and sends the signal to a door open/close means 4. The optical detection means 3 includes an optical sensor 3a (the first optical sensor) for detecting an instruction to open the door 2, and an optical sensor 3b (the second optical sensor) for detecting an instruction to close the door 2. When the operator holds his hand close to but not in contact with the optical sensor 3a in order to open the door 2, the optical sensor 3a detects it and sends a signal (the first signal) for opening the opening 1a. When the operator holds his hand close to but not in contact with the optical sensor 3b in order to close the door 2, the optical sensor 3b detects it and sends a signal (the second signal) for closing the opening 1a.

Upon reception of the signal from the optical detection means 3, the door open/close means 4 causes a driving means (not shown in the drawing) to move the door 2 and thereby controls the open/close of the opening 1a. In other words, upon reception of a signal (the first signal) that reflects the detection by the optical sensor 3a, the door open/close means 4 causes the driving means to move the door 2 to open the opening 1a, and upon reception of a signal (the second signal) that reflects the detection by the optical sensor 3b, the door open/close means 4 causes the driving means to move the door 2 to close the opening 1a.

Examples of the optical sensors can include an infrared sensor (particularly, a near-infrared sensor).

An aspect of the infrared sensor may include an infrared radiator for emitting infrared radiation, an infrared receptor for receiving reflected infrared radiation, and a detector for detecting changes in radiation reflected when the operator holds his hand or the like close to the detector (a reflection type sensor).

Another aspect of the infrared sensor may include an infrared radiator for emitting infrared radiation, an infrared receptor positioned in the path of the infrared radiation and receiving the infrared radiation emitted by the infrared radiator, and a detector for detecting the fact that the infrared radiation traveling toward the infrared receptor is blocked when the operator holds his hand or the like close to the path of the infrared radiation (a transmission type sensor).

This embodiment discloses an aspect in which the optical detection means 3 includes the optical sensor 3a for detecting an instruction to open the door 2 and the optical sensor 3b for detecting an instruction to close the door 2. Alternatively, an aspect of the optical detection means 3 may include only one optical sensor and the open/close of the door 2 may be controlled by switching the signal to send to the door open/close means 4 each time the operator holds his hand or the like close to the optical sensor.

For example, at the first time when the operator holds his hand close to the optical sensor, the optical detection means 3 may send a signal (the first signal) for opening the opening 1a, and at the second time when the operator holds his hand close to the optical sensor, the optical detection means 3 may send a signal (the second signal) for closing the opening 1a. After that, the optical detection means 3 alternately sends the first signal and the second signal each time the operator holds his hand or the like close to the optical sensor.

Alternatively, the first signal and the second signal may be the same signal, and the door open/close means 4 may switch opening and closing of the door 2 each time receiving the signal from the optical detection means 3.

Although FIG. 1 illustrates the case where the optical sensors 3a and 3b are set to the door 2, the location of the optical sensors may be determined according to the operability for the operator. The location is preferably a place with a low risk of malfunction.

As shown in FIG. 1, an aspect of the door 2 can include a plurality of plate-like units along the opening 1a and the plate-like units can move relatively to each other along the opening 1a, thereby opening and closing the opening 1a, for example.

In this embodiment, a far-infrared sensor may be used as the optical sensor. Alternatively, an electromagnetic sensor (e.g., a microwave sensor or a radio wave sensor) which detects electromagnetic waves may be used as the optical sensor.

### {Second embodiment}

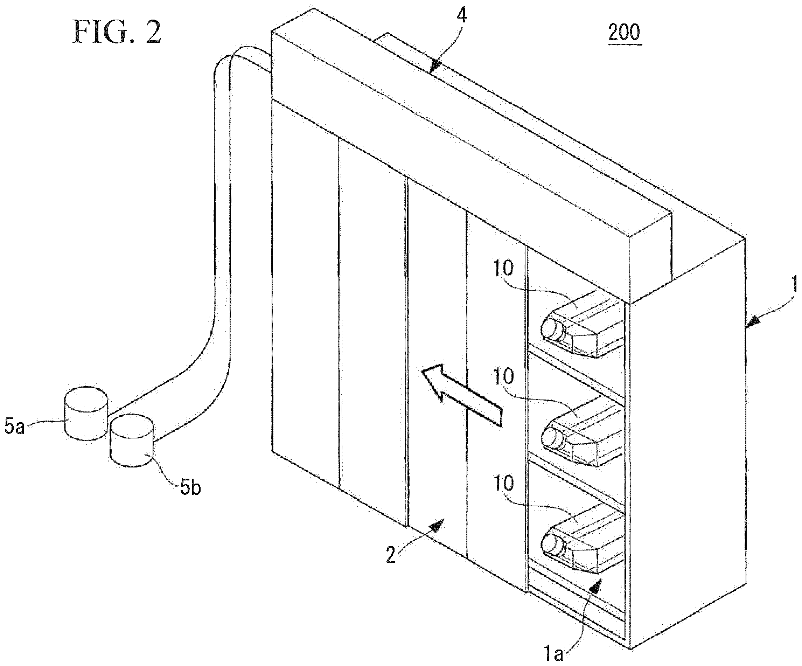

A cell culture apparatus according to the second embodiment of the present invention, which is an incubator 200 having the structure shown in FIG. 2, can maintain its inside at conditions (for example, temperature, humidity, and CO₂ concentration) suitable for cell culture and accommodate a culture container (cell culture container) 10 containing cells, thereby allowing for cell culture.

The incubator 200 differs from the first embodiment in that it includes a detection means 5 with a pedal, instead of the optical detection means 3. Aside from this, it has the same structure as the first embodiment.

When the operator steps on (pressures) the pedal with his foot or the like, the detection means 5 detects it and converts it to a signal and sends the signal to a door open/close means 4. The detection means 5 includes a pedal 5a (the first pedal) for detecting an instruction to open the door 2, and a pedal 5b (the second pedal) for detecting an instruction to close the door 2. The operator steps on the pedal 5a with his foot to open the door 2, and steps on the pedal 5b with his foot to close the door 2. When the operator steps on the pedal 5a with his foot in order to open the door 2, the pedal 5a detects it and sends a signal (the first signal) for opening the opening 1a. When the operator steps on the pedal 5b with his foot in order to close the door 2, the pedal 5b detects it and sends a signal (the second signal) for closing the opening 1a.

Upon reception of the signal from the detection means 5, the door open/close means 4 causes a driving means to move the door 2 and thereby controls the open/close of the opening 1a. In other words, upon reception of a signal (the first signal) that reflects the detection by the pedal 5a, the door open/close means 4 causes the driving means to move the door 2 to open the opening 1a, and upon reception of a signal (the second signal) that reflects the detection by the pedal 5b, the door open/close means 4 causes the driving means to move the door 2 to close the opening 1a.

This embodiment discloses an aspect in which the detection means 5 includes the pedal 5a for detecting an instruction to open the door 2 and the pedal 5b for detecting an instruction to close the door 2. Alternatively, an aspect of the detection means 5 may include only one pedal and the open/close of the door 2 may be controlled by switching the signal to send to the door open/close means 4, each time the operator steps on the pedal with his foot or the like.

For example, at the first time when the operator steps on the pedal, the detection means 5 may send a signal (the first signal) for opening the opening 1a, and at the second time when the user steps on the pedal, the detection means 5 may send a signal (the second signal) for closing the opening 1a. After that, the detection means 5 alternately sends the first signal and the second signal each time the operator steps on the pedal.

Alternatively, the first signal and the second signal may be the same signal, and the door open/close means 4 may switch opening and closing of the door 2 each time receiving the signal from the detection means 5.

In the above embodiments, the driving means for the door open/close means 4 may include, for example, a linear-motion mechanism with a ball screw, rotate the ball screw by the use of a motor or the like, and convert rotational motion into linear motion to move the door 2 along a guide rail or the like.

Alternatively, the driving means for the door open/close means 4 may include, for example, a pulley and a belt (a wire or a chain), add rotational power to the pulley with the use of a motor or the like, and convert rotational motion into linear motion through the belt (a wire or a chain) to move the door 2 along a guide rail or the like.

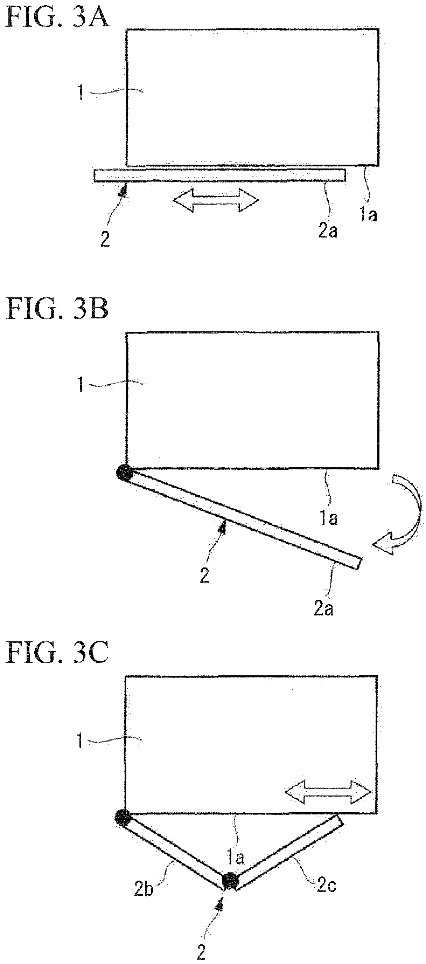

In the above embodiments, as shown in FIG. 1, an aspect of the door includes a plurality of plate-like units along the opening and the plate-like units move relatively to each other along the opening, thereby opening and closing the opening 1a, for example. Alternatively, the door 2 as shown in any of FIGS. 3A to 3C may be used, for example. FIG. 3A illustrates the case where a single plate-like unit 2a moves (slides) along the opening. FIG. 3B illustrates the case where a single plate-like unit 2a swings about one of its sides and moves relatively to the opening. FIG. 3C illustrates the case where plate-like units 2b and 2c are folded and move relatively to the opening.

In the above embodiments, the optical detection means 3 (or the detection means 5) may send signals to the door open/close means 4 either in a hardwired or wireless manner.

The present invention can provide a cell culture apparatus including: a body including a space for accommodating a culture container, and an opening through which the culture container is taken in and out; a door for opening and closing the opening; an detection means that includes a sensor for detecting an instruction to open and close the opening from an operator, and converts the detection by the sensor to a signal and sends the signal; and a door open/close means that receives the signal from the detection means and controls open/close of the opening by the door.

### {Reference Signs List}

- 1: body
- 2: door
- 3a, 3b: optical sensor
- 4: door open/close means
- 5a, 5b: pedal
- 100, 200: incubator (cell culture apparatus)

## Claims

1. A cell culture apparatus comprising:
a body including a space for accommodating a culture container, and an opening through which the culture container is taken in and out;
a door for opening and closing the opening;
an optical detection means that includes an optical sensor for optically detecting an instruction to open and close the opening from an operator, and converts the detection by the optical sensor to a signal and sends the signal; and
a door open/close means that receives the signal from the optical detection means and controls open/close of the opening by the door.

2. The cell culture apparatus according to Claim 1, wherein
the optical detection means includes a first optical sensor that optically detects an instruction to open the opening, and a second optical sensor that optically detects an instruction to close the opening,
the optical detection means sends a first signal for opening the opening in response to the detection by the first optical sensor and sends a second signal for closing the opening in response to the detection by the second optical sensor, and
the door open/close means opens the opening with the door upon reception of the first signal, and closes the opening with the door upon reception of the second signal.

3. The cell culture apparatus according to Claim 1, wherein
the optical detection means includes a single optical sensor,
the optical detection means alternately sends a first signal for opening the opening and a second signal for closing the opening each time the optical sensor performs detection, and
the door open/close means opens the opening with the door upon reception of the first signal, and closes the opening with the door upon reception of the second signal.

4. The cell culture apparatus according to any one of Claims 1 to 3, wherein the optical sensor is an infrared sensor.

5. A cell culture apparatus comprising:
a body including a space for accommodating a culture container and an opening through which the culture container is taken in and out;
a door for opening and closing the opening;
an detection means that includes a pedal for detecting an instruction to open and close the opening from an operator, and converts the detection by the pedal to a signal and sends the signal; and
a door open/close means that receives the signal from the detection means and controls open/close of the opening by the door.

6. The cell culture apparatus according to Claim 5, wherein
the detection means includes a first pedal for detecting an instruction to open the opening, and a second pedal for detecting an instruction to close the opening,
the detection means sends a first signal for opening the opening in response to the detection by the first pedal, and a second signal for closing the opening in response to the detection by the second pedal, and
the door open/close means opens the opening with the door upon reception of the first signal, and closes the opening with the door upon reception of the second signal.

7. The cell culture apparatus according to Claim 5, wherein
the detection means includes a single pedal,
the detection means alternately sends a first signal for opening the opening and a second signal for closing the opening each time the pedal performs detection, and
the door open/close means opens the opening with the door upon reception of the first signal, and closes the opening with the door upon reception of the second signal.

8. A cell culture apparatus comprising:
a body including a space for accommodating a culture container, and an opening through which the culture container is taken in and out;
a door for opening and closing the opening;
an detection means that includes a sensor for detecting an instruction to open and close the opening from an operator, and converts the detection by the sensor to a signal and sends the signal; and
a door open/close means that receives the signal from the detection means and controls open/close of the opening by the door.
